# EUROPEAN PATENT APPLICATION

(11) **EP 1 482 056 A2**
(43) Date of publication of application: **01.12.2004**
(21) Application number: 04012639.3
(22) Date of filing: 27.05.2004
(51) Int. Cl.: C12Q 1/00, G01N 33/487

(54) **Biosensor**

(30) Priority: 28.05.2003 JP 2003151067
(71) Applicant: MATSUSHITA ELECTRIC INDUSTRIAL CO., LTD., Kadoma-shi, Osaka 571-8501 (JP)
(72) Inventor: Ikeda, Shin, Osaka 576-0022 (JP); Nakaminami, Takahiro, Toyonaka-shi, Osaka 560-0033 (JP); Yoshioka, Toshihiko, Osaka 573-0035 (JP); Kuwabata, Susumu, Suita-shi Osaka 565-0872 (JP)
(74) Representative: Leson, Thomas Johannes Alois, Dipl.-Ing.

(57) **Abstract**

The present invention is to provide a biosensor capable of determining the concentration of a substrate with higher precision. The biosensor comprises an electrically insulating substrate, a measuring system, a reagent layer including at least an oxidoreductase and an electron mediator, and the biosensor has a member for elimination of interfering compounds including: a redox agent which functions as an oxidant for oxidizing interfering compounds in a sample, and a carrier for immobilizing the redox agent.

## Description

### BACKGROUND OF THE INVENTION

The present invention relates to a biosensor that can rapidly determine a substrate concentration in a sample with high precision and in a simple manner.

In recent years, various types of biosensors utilizing a specific catalytic activity possessed by an enzyme have been developed for determining a sugar such as sucrose or glucose. The assay of glucose is now described as an example of the assay of a substrate in a sample solution. A commonly known electrochemical assay of glucose involves the use of glucose oxidase (EC1.1.3.4, hereinafter abbreviated as "GOD") and an oxygen electrode or hydrogen peroxide electrode.

GOD selectively oxidizes β-D-glucose serving as a substrate to D-glucono-δ-lactone with the use of oxygen as an electron mediator. During the oxidation reaction by GOD in the presence of oxygen, oxygen is reduced to hydrogen peroxide. A decrease of oxygen is measured by an oxygen electrode. Alternatively, an increase of hydrogen peroxide is measured by a hydrogen peroxide electrode. The decreased amount of oxygen and the increased amount of hydrogen peroxide are proportional to the amount of glucose in a sample solution and therefore the concentration of glucose can be determined from the decreased amount of oxygen or the increased amount of hydrogen peroxide.

As can be gathered from the reaction process, the above-described method is accompanied by a defect that the result is largely affected by the concentration of oxygen contained in a sample solution. In addition to that, the absence of oxygen in a sample solution makes the measurement impossible. In view of this, a new type of glucose sensor which utilizes, as the electron mediator, an organic compound or metal complex such as potassium ferricyanide, a ferrocene derivative or a quinone derivative instead of oxygen has been developed.

In a sensor of this type, a reduced form of the electron mediator generated from an enzyme reaction is oxidized on an electrode, and the concentration of glucose contained in the sample solution is determined from the change of the oxidation current level. With the use of such organic compound or metal complex as the electron mediator instead of oxygen, it is possible to accurately carry a known amount of GOD and the electron mediator on the electrode in the stable conditions to form a reagent layer. In this case, the reagent layer can be integrated with an electrode system in a semi-dried condition.

Much attention has recently been paid to a disposable glucose sensor based on such technique as disclosed by, for example, the specification of United States Patent No. 5120420. In a disposable glucose sensor, the concentration of glucose is measured by a meter device in a very simple way of just introducing a sample solution into a detachable sensor connected to the meter device. The technique like this can be applied not only to the determination of the concentration of glucose but also to the determination of the concentration of other substrate contained in the sample solution.

In the measurement using the sensor mentioned above, a reduced form of electron mediator is oxidized on a working electrode, during which an oxidation current flows. The concentration of a substrate can be determined based on the oxidation current level. In the case of the sample being blood or fruit juice, easily-oxidizable interfering compounds such as ascorbic acid and uric acid contained in the sample solution are also oxidized on the working electrode with the reduced form of electron mediator. The oxidation reaction of the easily-oxidizable interfering compounds may provide a result with a margin of error in some cases. Moreover, the contact of an oxidized form of electron mediator with the easily-oxidizable interfering compounds produces a reduced form of electron mediator regardless of an enzyme reaction, which could provide a result with a margin of error.

Samples to be measured by biosensors normally contain interfering compounds that can affect the measurement of a specific component. In order to reduce influences of the interfering compounds, Japanese Patent No. 3102613, for example, proposes a technique in which interfering compounds are oxidized by an enzyme in the upstream portion of a biosensor. United States Patent No. 6340428 also proposes a technique in which interfering compounds are oxidized on an electrode in the upstream portion of a biosensor. None of the above techniques, however, provide a complete solution for dealing with the result of a measurement with a margin of error.

### BRIEF SUMMARY OF THE INVENTION

In view of the above, the object of the present invention is to provide a biosensor capable of rapidly determining the concentration of a substrate in a sample solution with high precision and in a simple manner without influences of easily-oxidizable interfering compounds contained in the sample solution.

In order to solve the above-described problems, the present invention provides a biosensor comprising an electrical insulating substrate, a measurement system and a reagent layer comprised of at least an oxidoreductase and an electron mediator,
wherein the biosensor further comprises a member for elimination of interfering compounds including: a redox agent which functions as an oxidant for oxidizing interfering compounds contained in a sample, and a carrier for immobilizing the redox agent.

The biosensor preferably further comprises a sample-supplying path composed of the substrate, a spacer member and a cover member.

The member for elimination of interfering compounds is located in a portion with which a sample can be in contact when the sample is supplied into the biosensor. More specifically, the reagent layer and the member for elimination of interfering compounds are located within the sample-supplying path, and the member for elimination of interfering compounds is preferably disposed upstream from the reagent layer.

The sample is preferably a biological sample and the interfering compounds are an easily-oxidizable compounds.

The redox agent is preferably a ferricyanide.

The carrier preferably comprises an ion-exchanging polymer.

The measurement system preferably comprises a working electrode and a counter electrode which are formed on the substrate.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWING

FIG. 1 is a perspective view of a disassembled biosensor used in one embodiment of the present invention.

FIG. 2 is a sectional view of the glucose sensor taken on line X-X of FIG. 1.

### DETAILED DESCRIPTION OF THE INVENTION

In order to solve the problems noted above, the present invention comprises a biosensor comprising an electrically insulating substrate, a measurement system and a reagent layer comprised of at least an oxidoreductase and an electron mediator, characterized in that the biosensor further comprises a member for elimination of interfering compounds including: a redox agent which functions as an oxidant for oxidizing interfering compounds contained in a sample, and a carrier for immobilizing the redox agent.

The term "interfering compound" used herein means a substance which is present with a compound to be measured in a sample and affects sensor's response signal to the compound to be measured. In the case of determining the concentration of a compound by oxidizing an electron mediator with the use of blood as the sample, for example, the interfering compounds are mainly ascorbic acid, uric acid, acetaminophen and the like. These substances are easily-oxidizable compounds.

In the biosensor according to the present invention, the member for elimination of interfering compounds, which includes a redox agent which functions as an oxidant for oxidizing such interfering compounds contained in the sample and a carrier for immobilizing the redox agent, treats a sample such as biological sample or fruit juice containing easily-oxidizable compounds such as ascorbic acid and uric acid. The member for elimination of interfering compounds also prevents the influences of the interfering compounds on the sensor response.

As described above, the contact of the oxidized form of electron mediator carried on the sensor electrode system with the easily-oxidizable compound may produce a reduced form of electron mediator regardless of an enzyme reaction. The present invention utilizes this property and reduces the influences of the interfering compounds. For instance, when a solution containing ascorbic acid contacts ferricyanide, the oxidized form of electron mediator, redox reaction occurs between ferricyanide and ascorbic acid, whereby ferricyanide is reduced into ferrocyanide and ascorbic acid is oxidized into an irreversible product. The dispersion or diffusion of the ferrocyanide in the sensor's electrode system will provide a response value having a margin of error.

In the biosensor according to the present invention, on the other hand, ferricyanide ion, an example of the redox agent which works as an oxidant for oxidizing the interfering compounds is electrostatically immobilized to a cationic polymer membrane, which constitutes the carrier in the member for elimination of interfering compounds. For instance, Oyama et al.: Anal. Chem., 58(4). 979-981(1986) discloses an example of the immobilizing technique. This reduces the influences of the easily-oxidizable compounds described above. Once ascorbic acid is oxidized, it becomes stable and its reducing ability is greatly reduced. Accordingly, the influences on the sensor's electrode reaction are also greatly reduced.

The oxidoreductase contained in the reagent layer can be appropriately selected according to the substrate contained in a sample. Examples of the oxidoreductase for use include fructose dehydrogenase, glucose oxidase, glucose dehydrogenase, alcohol oxidase, lactate oxidase, cholesterol oxidase, xanthine oxidase and amino acid oxidase.

As the electron mediator, there are potassium ferricyanide, p-benzoquinone, phenazine methosulfate, methylene blue, a ferrocene derivative, an osmium complex, a ruthenium complex and the like. Even when oxygen is used as the electron mediator, the current response can be obtained. They may be used singly or in combination of two or more. It is to be noted that the term "electron mediator" used in this specification denotes a material which exchanges electrons with the enzyme.

Particularly when the measurement system is an optical type, the electron mediator can be a dye. Potassium ferricyanide and phenazine methosulfate listed above can also be used as the dye.

The member for elimination of interfering compounds, which is the main feature of the present invention, includes a redox agent which functions as an oxidant for oxidizing interfering compounds and a carrier for immobilizing the redox agent. In the case of the interfering compound being ascorbic acid, an agent having a higher standard oxidation-reduction (redox) potential than ascorbic acid, which has a standard oxidation-reduction potential of 0.058 V, is preferably used as the redox agent which functions as an oxidant for oxidizing interfering compounds.

More preferably, the redox agent which functions as an oxidant for oxidizing an interfering compound and the electron mediator comprise the same compound. Thereby, the structural convenience and simplicity of the sensor can be improved.

The carrier for immobilizing the redox agent preferably comprises an ion-exchanging polymer. Due to electrostatic interactions, the redox agent is immobilized on the ion-exchanging polymer. For this reason, a cationic ion exchanging polymer is preferably used when an anionic redox agent is employed. For example, polyvinyl pyridine or poly(N,N-dimethylaniline) can be used as the carrier for immobilizing ferricyanide ion. When a cationic redox agent is used, on the other hand, an anionic ion exchanging polymer is preferably employed. As an example, ferrocenyl methyl trimethylammonium (Fc-CH₂-NMe₃) can be immobilized on perfluorocarbon sulfonic acid (Nafion made by E. I. Du Pont de Nemours & Co. Inc., USA).

Further, the carrier for immobilizing the redox agent may be a carrier which can immobilize the redox agent by covalent or coordinate bonding. Polylysine, for example, has amino residues and therefore a redox agent having an amino group can be immobilized thereon by covalent bonding with the use of glutaraldehyde as a crosslinking agent. Polyvinylimidazole has an imidazole group which functions as a ligand and therefore a metal complex such as an osmium complex (Os(bpy)₂Cl) can be immobilized thereon.

By immobilizing the reagent layer on the working electrode, the enzyme or electron mediator can be insolubilized. The immobilization is preferably done by crosslinking or absorption. Alternatively, components of the reagent layer may be mixed with electrode materials.

The working electrode may be made of any conductive material that is not oxidized when the electron mediator is oxidized. The electrode system is preferably produced by screen printing, sputtering, vapor deposition or the like.

In the following, the present invention is described using examples, but it is to be understood that the present invention is not limited to them.

### EXAMPLE 1

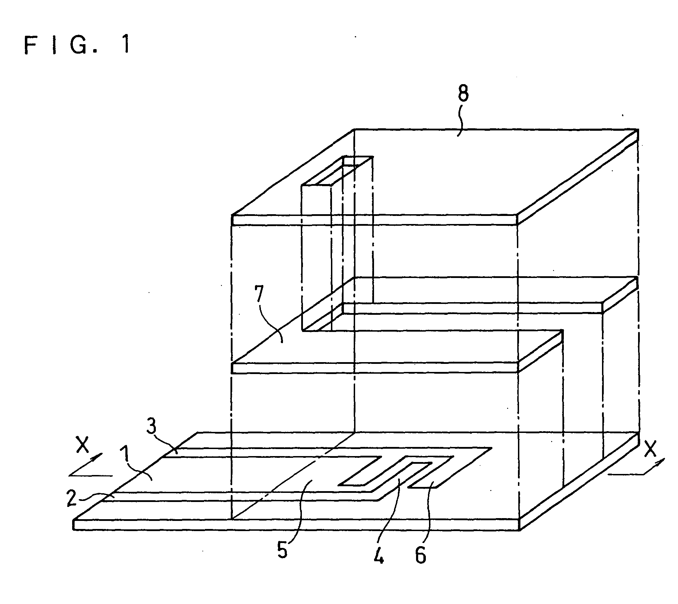

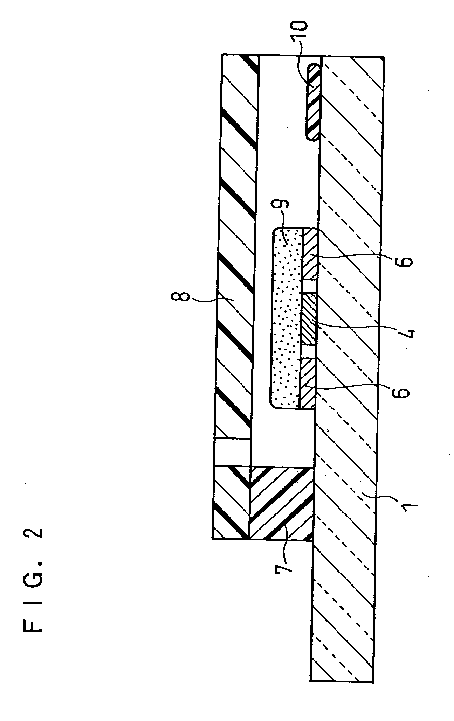

As one embodiment of the biosensor in accordance with the present invention, a glucose sensor having a structure as shown in FIGs. 1 and 2 was produced. FIG. 1 is a perspective view of a disassembled glucose biosensor without a reagent layer and the like. FIG. 2 is a sectional view taken on line X-X of FIG. 1.

A stainless steel plate having an opening of two dimensional shape, which corresponds to leads 2 and 3 and a working electrode 4 and a counter electrode 6 shown in FIG. 1, was put tightly on an electrical insulating substrate 1 made of polyethylene terephthalate. Palladium was sputtered on the masked substrate 1 as described above to form the leads 2 and 3 and the working electrode 4 and the counter electrode 6. Then, the stainless steel plate was removed. At the same time, an insulating portion 5 was formed.

Subsequently, an aqueous solution of carboxymethyl cellulose (CMC) was dropped onto an electrode system composed of the working electrode 4 and the counter electrode 6, which was then dried to form a CMC layer. An aqueous solution containing GOD as the enzyme and potassium ferricyanide as the electron mediator was dropped onto the CMC layer, followed by drying to form a reagent layer 9.

In order to facilitate the supply of a sample solution into the reagent layer, a lecithin layer (not shown in the figures) was formed on the reagent layer by spreading a toluene solution of lecithin onto the reagent layer from a sample-supplying path inlet, followed by drying. Although toluene was used to form the lecithin layer in this example, other organic solvent may be used.

A solution (ternary solvent mixture of water, methanol and 2-propanol) of polyvinylpyridine (cationic polymer) was dropped in an appropriate amount onto a portion on the substrate 1 corresponding to a section regulated by combining a cover 8 and a spacer 7, which was then air-dried to form a polymer layer serving as the carrier. This polymer layer was immersed in an aqueous solution containing 0.2 mM of potassium ferricyanide for 1 hour so as to effect an ion exchange reaction and to condense and immobilize ferricyanide ion within the polymer layer. Thereby, a member for elimination of interfering compounds 10 was formed.

The concentration of ferricyanide ion contained in the polymer layer determined from a cyclic voltammogram was 2000 to 3000 times higher than that of the solution. The substrate having the member for elimination of interfering compounds produced in the above manner and the spacer/cover were attached in such a positional relationship shown by the dashed line with a dot in FIG. 1 to give a glucose sensor according to the present invention.

The produced glucose sensor was connected to a measuring device, and an aqueous solution of glucose (360 mg/dl) was then fed thereinto. After a certain period of time, a voltage of 500 mV was applied between the working electrode 4 and the counter electrode 6. A current level was measured 5 seconds after the application of the voltage. Ferricyanide ion, glucose and GOD reacted in the solution. Specifically, glucose was oxidized into gluconolacton, and ferricyanide ion was reduced into ferrocyanide ion. The produced ferrocyanide ion was oxidized and thereby a current response was obtained. The current response was proportional to the concentration of glucose in the sample solution.

Another measurement was performed in the same manner as above except for feeding an aqueous solution of glucose containing 10 mg/dl of ascorbic acid (360 mg/dl). The result obtained from this measurement was compared to that obtained from a comparative measurement performed in the same manner as above using a sensor without the member for elimination of interfering compounds. It was found from the comparison that the increase of the sensor response that would otherwise occur due to the addition of ascorbic acid was greatly reduced in the sensor having the member for elimination of interfering compound.

Although, in EXAMPLE 1, the same material was used as both the redox agent which functions as an oxidant for oxidizing the interfering compounds and the electron mediator, the present invention is not limited to the above, and the redox agent and the electron mediator may be two different materials.

The voltage applied to the electrode system in order to obtain the current response is also not limited to 500 mV which was used in EXAMPLE 1. The applied voltage may be any value as long as a variation of the electric signal is observed and the electron mediator is oxidized.

Moreover, the electrode system, the lead/terminal shown in this example is merely an example, and the shape, arrangement and number thereof are not limited to the above.

### EXAMPLE 2

A biosensor was produced in the same manner as in EXAMPLE 1, except that the substrate 1 and the cover 8 were made of glass. In this example, the electrodes and leads were shaped without the printing of the insulating paste.

An aqueous solution of carboxymethyl cellulose (CMC) was dropped onto a substrate 1, followed by drying, to form a CMC layer. On the CMC layer was dropped an aqueous solution containing GOD as the enzyme and 1-methoxy-5-methyl-phenazinium as the electron mediator, which was then dried to form a reagent layer.

In order to facilitate the supply of a sample solution into the reagent layer, a lecithin layer was formed on the reagent layer by spreading a toluene solution of lecithin onto the reagent layer from a sample-supplying path inlet, followed by drying.

Subsequently, a solution of Nafion (anionic polymer) was dropped in an appropriate amount onto a portion on the substrate 1 corresponding to a section regulated by combining a cover 8 and a spacer 7, which was then air-dried to form a polymer layer serving as the carrier. This polymer layer was immersed in an aqueous solution containing 0.2 mM of 1-methoxy-5-methyl-phenazinium for 1 hour to condense and immobilize 1-methxy-5-methyl-phenazinium ion in the polymer layer.

The substrate having the member for elimination of interfering compounds formed in the above manner and the spacer/cover were attached in such a positional relationship shown by the dashed line with a dot in FIG. 1 to give a glucose sensor according to the present invention.

An aqueous solution of glucose (360 mg/dl) was fed into the produced glucose sensor. After a certain period of time, light with a wavelength of 620 nm was applied vertically to the substrate 1 and the cover 8, and its absorbance was measured by an absorptiometer. After a certain period of time, the absorbance was again measured. A decrease in absorbance with time was observed. This is because 1-methxy-5-methyl-phenazinium, glucose and GOD reacted, specifically, glucose was oxidized and 1-methxy-5-methyl-phenazinium was reduced. The degree of the decrease in absorbance was proportional to the concentration of glucose contained in the sample solution.

Another measurement was performed in the same manner as above except for feeding an aqueous solution of glucose containing 10 mg/dl of ascorbic acid (360 mg/dl), and a decrease in absorption similar to that observed in the case of using the glucose solution without ascorbic acid was observed.

For comparison, a sensor for comparison was produced in the same manner as above except that the member for elimination of interfering compounds was not formed, and the absorbance thereof was then measured in the same manner as above. From the comparison between the obtained absorbance and that of the above, it was clear that a decrease in absorbance of the sensor for comparison was greater. Presumably, this is because 1-methxy-5-methyl-phenazinium used as the electron mediator was directly reduced by ascorbic acid without the enzyme reaction with glucose. The foregoing has revealed that, even in an optical sensor, a margin of error that would otherwise occur by the addition of ascorbic acid was greatly reduced if the sensor has the member for elimination of interfering compounds.

As described above, according to the biosensor in accordance with the present invention, it is possible to determine the concentration of a substrate in a sample with high precision and in a simple and rapid manner.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

The present invention is to provide a biosensor capable of determining the concentration of a substrate with higher precision. The biosensor has a member for elimination of interfering compounds including: a redox agent which functions as an oxidant for oxidizing interfering compounds in a sample, and a carrier for immobilizing the redox agent.

## Claims

1. A biosensor comprising, an electrical insulating substrate; a measurement system, a reagent layer including at least an oxidoreductase and an electron mediator, and a member for elimination of interfering compounds,
said member for elimination of interfering compounds comprising:
a redox agent which functions as an oxidant for oxidizing interfering compounds in a sample; and
a carrier for immobilizing said redox agent.

2. The biosensor in accordance with claim 1, further comprising a sample-supplying path comprising said substrate, a spacer and a cover.

3. The biosensor in accordance with claim 1, wherein said member for elimination of interfering compounds is located upstream from the reagent layer.

4. The biosensor in accordance with claim 1, wherein said sample is a biological sample and said interfering compounds is an easily-oxidizable compound.

5. The biosensor in accordance with claim 1, wherein said redox agent is a ferricyanide.

6. The biosensor in accordance with claim 1, wherein said carrier comprises an ion-exchanging polymer.

7. The biosensor in accordance with claim 1, wherein said measurement system comprises a working electrode and a counter electrode which are formed on said substrate.

8. The biosensor in accordance with claim 1, wherein said electron mediator and said redox agent comprise the same compound.
